# EUROPEAN PATENT SPECIFICATION

(11) **EP 3 973 101 B1**
(45) Date of publication and mention of the grant of the patent: **24.06.2026**
(21) Application number: 20809099.3
(22) Date of filing: 05.05.2020
(51) Int. Cl.: D21C 1/04, D21C 1/02, C08B 1/00, C08B 16/00, D21C 11/02, D21C 3/26

(54) **A METHOD FOR EXTRACTING HYDROLYSATE, A BATCH COOKING SYSTEM AND A HYDROLYSATE EXTRACTING ARRANGEMENT**
VERFAHREN UND VORRICHTUNG ZUM EXTRAHIEREN VON HYDROLYSAT, BATCHKOCHSYSTEM UND ANORDNUNG ZUR HYDROLYSATEXTRAKTION
PROCÉDÉ D'EXTRACTION D'HYDROLYSAT, SYSTÈME DE CUISSON PAR LOTS ET AGENCEMENT D'EXTRACTION D'HYDROLYSAT

(30) Priority: 22.05.2019 SE 1950606
(43) Date of publication of application: 30.03.2022
(73) Proprietor: VALMET AB, 851 94 Sundsvall (SE)
(72) Inventor: LANDMAN, Hunphrey, 1200 Nelspruit (ZA); MINNAAR, Susanna, 0081 Pretoria (ZA); LAMMI, Lari, 282 20 Pori (FI); KARVONEN, Juoni, 284 50 Vanha-Ulvila (FI); COETZEE, Berdine, 0081 Pretoria (ZA); WAUTS, Johann, 1201 Nelspruit (ZA)
(74) Representative: Novagraaf Group
(86) International application number: PCT/SE2020/050454
(87) International publication number: WO 2020/236061

(56) References cited:
- EP-A1- 3 018 251
- EP-A1- 3 026 171
- WO-A1-2007/090926
- WO-A1-2011/110746
- WO-A1-2016/191503
- WO-A1-2017/142445
- WO-A1-95/20065
- CN-B- 103 757 961
- US-A- 5 676 795
- US-A1- 2011 056 637

## Description

### TECHNICAL FIELD

The present invention relates to a method for extracting hydrolysate in a batch cooking process for producing pulp. It furthermore relates to a batch cooking system for producing pulp and to a hydrolysate extracting arrangement.

### BACKGROUND

In some pulp production, for example in dissolving pulp production, lignocellulose raw material is first subject to a hydrolysis for removing unwanted hemicelluloses and then an alkaline cooking is performed for removing lignin. The hydrolysis in a batch cooking system is normally performed by adding steam to the lignocellulose raw material. During the hydrolysis the hemicellulose, mainly C5 sugars, are degraded and released from the wood. If the sugars should be extracted and taken care of a hydrolysate comprising the dissolved sugars needs to be removed from a vessel where the hydrolysis is performed before the pH is changed and an alkaline cooking is performed. In the alkaline cooking process, the sugars are broken-down to non-valuable components.

During production of dissolving pulp from lignocellulosic material it is generally desirable to remove hemicellulose in order to obtain a pulp with a high content of alpha-cellulose. This is also done for various other kinds of specialty chemical pulp.

The removal is performed by a prehydrolysis step where a treatment liquor such as steam or water is introduced, and hemicellulose is hydrolysed from the wood chips during acid conditions. Afterwards, the slurry thus created is neutralized by an alkaline neutralizing liquor to produce a neutralized hydrolysate. Hemicellulose is broken down by the contact with alkali into smaller components and is removed from the slurry in subsequent washing stages during the pulp production.

In some applications, it is now desirable to preserve the hemicellulose in hydrolysate and remove it from the slurry for further use in other fields of industry such as the food industry, livestock industry, or biofuels. However, only a small part of available hemicellulose is removed using prior art methods. It is also difficult to keep the hemicellulose separate from the alkali added to the hydrolysate and used for neutralization, and to remove and preserve hemicellulose without lowering the efficiency of the pulp process.

Since the economic value of the finished pulp is at the moment significantly higher than that of the removed hemicellulose, any commercially viable process for extracting and removing hemicellulose cannot be allowed to impair the pulping process.

There is therefore a need for a method for extracting hemicellulose from lignocellulose material that takes the above considerations and drawbacks into account and that enables removal of hemicellulose in a more efficient way while at the same time having a minimal impact on the process for producing pulp.

Steam hydrolysis only in batch cooking will give very low sugar yield to hydrolysate as the steam condensate volume is too low to access most of the dissolved sugars, which can be still inside the chips after the hydrolysis. Water hydrolysis of liquid phase hydrolysis is suitable if higher and controllable sugar recoveries are sought for. In such process the chips are heated up with steam and after reaching the target hydrolysis temperature, or at a specific P-factor, preheated liquid is added to the digesters and the dissolved sugars will move to the liquid phase to be more easily removed from the digester and the recovery of the sugars is improved compared to e.g. steam hydrolysis. However, the addition of large volumes of water may result in low dry solids in the hydrolysate resulting in additional evaporation need in hydrolysate evaporation and also in higher water flow to kraft liquor evaporation.

EP3026171 describes a displacement batch cooking process comprising a displacement phase using a temperature gradient in the displacement liquor used.

EP3018251 describes a displacement batch cooking process recovering a first treatment liquid in a displacement batch pulping process by draining off the first treatment liquid from digester and using a displacement liquid.

WO2017/142445 describes a method for recovering concentrated hydrolysate after hydrolysis of cellulose material in a batch digester.

WO2007/090926 describes a method for production of carbohydrates in connection with pulp production.

### SUMMARY

An object of the present invention is to provide an effective method for extracting hydrolysate in a pulp production system where the amount of added fresh water can be limited.

This is achieved by a method for extracting hydrolysate and by a batch cooking system and by a hydrolysate extracting arrangement according to the independent claims 1,8 and 14.

Hereby, by separating the first part of the hydrolysate for further use, only the purest sugar solution is extracted. A second part of the hydrolysate is instead used for displacing hydrolysate in a next batch cooking process. Hereby a more efficient hemicellulose extraction can be achieved both because less water needs to be evaporated and because there is a decreased risk of inclusion of alkaline in the extracted hydrolysate. The second part of the extracted hydrolysate may possibly contain small amounts of impurities, e.g. alkali and Na-ions and by not using this second part for extraction of hemicellulose but instead use the second part for displacing hydrolysate in a next batch the problem of possible impurities, e.g. alkali and Na-ions in the extracted hydrolysate is much decreased or even eliminated. Furthermore, by recirculating parts of the hydrolysate the volume of fresh water needed is limited which is suitable for the pulp processing, e.g. less evaporation needs, and for water saving reasons.

In one embodiment of the invention the method comprises the further step:
- recirculating said second part of the hydrolysate from the hydrolysate recirculation tank to the batch cooking vessel for displacing a first part of a hydrolysate from the batch cooking vessel in a next batch cooking process performed in the batch cooking vessel.

In one embodiment of the invention the method further comprises the step of extracting sugar from the hydrolysate in the hydrolysate extraction tank.

In one embodiment of the invention the method further comprises the step of performing alkaline cooking in the batch cooking vessel after the displacing of the first and second parts of the hydrolysate.

In one embodiment of the invention the batch cooking process is a process for producing dissolving pulp.

In one embodiment of the invention the step of performing acid hydrolysis of the lignocellulose raw material in the batch cooking vessel comprises steam hydrolysis and/or liquid hydrolysis.

In one embodiment of the invention the step of receiving the second part of the hydrolysate in at least one hydrolysate recirculation tank comprises receiving the second part of the hydrolysate in at least two hydrolysate recirculation tanks alternately such that one of the hydrolysate recirculation tanks can be cleaned while the other is used.

In one embodiment of the invention said batch cooking system further comprises an alkaline neutralization liquor tank connected to an inlet of the batch cooking vessel via an alkaline neutralization liquor conduit and an alkaline neutralization liquor pump provided in connection with said alkaline neutralization liquor conduit, wherein the control system further is connected to said alkaline neutralization liquor pump and is configured for controlling said alkaline neutralization liquor pump to add alkaline neutralization liquor from the alkaline neutralization liquor tank into the batch cooking vessel for displacing at least a part of the second part of the hydrolysate.

In one embodiment of the invention said batch cooking vessel is a pressurized vessel and said batch cooking system further comprises a hydrolysis arrangement connected to the batch cooking vessel, which hydrolysis arrangement is configured for adding a fluid to the batch cooking vessel for performing a hydrolysis of a lignocellulose raw material provided in the batch cooking vessel.

In one embodiment of the invention the hydrolysis arrangement is configured for adding steam and/or liquid to the batch cooking vessel for performing the hydrolysis.

In one embodiment of the invention the batch cooking system is a system for producing dissolving pulp.

In one embodiment of the invention the hydrolysate extracting arrangement comprises at least two hydrolysate recirculation tanks such that they can be used alternately.

### BRIEF DESCRIPTION OF THE DRAWINGS

Figure 1 shows schematically a batch cooking system according to one embodiment of the invention.
Figure 2 is a flow chart of a method according to one embodiment of the invention.

### DETAILED DESCRIPTION OF EMBODIMENTS

The method according to the invention is suitable for use with any process where lignocellulosic material undergoes hydrolysis as part of a pulping process.

The term lignocellulosic material is used herein to mean materials containing lignin, cellulose and hemicellulose. One example of such materials is wood, others include other agricultural or forestry wastes. The lignocellulosic material is commonly divided into small pieces, chips or fragments before the pulping process is initiated.

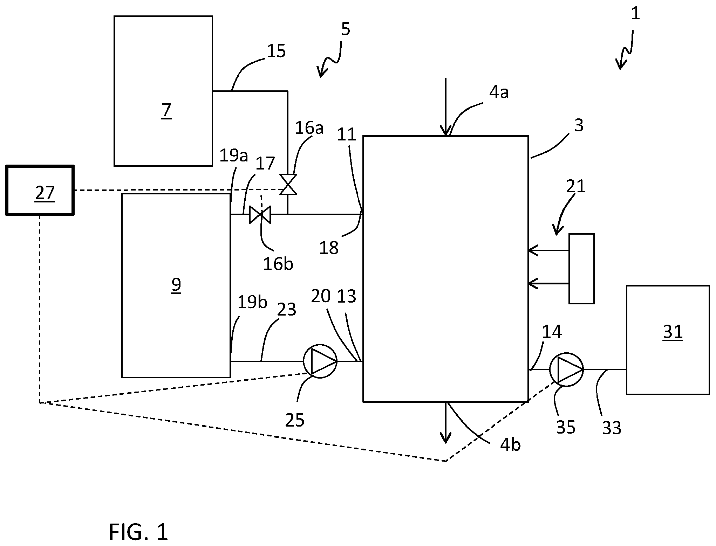

The lignocellulosic material is supplied to a batch cooking vessel 3 in a batch cooking system 1. One embodiment of a batch cooking system 1 is schematically shown in Figure 1. A treatment liquor is added to the batch cooking vessel 3 so that hydrolysis of the lignocellulosic material can be performed. A hydrolysis arrangement 21 is shown schematically in Figure 1, which hydrolysis arrangement 21 is configured to add treatment liquor for performing hydrolysis. The treatment liquor may be a gas or a liquid that serves to create acidic conditions in the batch cooking vessel 3. In some embodiments, the gas or liquid itself has a low pH, but in some embodiments the gas or liquid is water or condensate in the form of steam or fluid and the acidic conditions are created by the lignocellulosic material itself releasing acidic substances when in contact with the treatment liquor at elevated temperatures. This is well known within the art and will not be described in detail herein.

Suitably, steam and/or water is supplied as treatment liquor for the hydrolysis. Suitably, a combination is used where steam is first supplied and water added later. The steam will extract hemicellulose and other substances from the lignocellulosic material in a more efficient way than a liquid. Furthermore, this makes the process more economical due to the possibility of heating the water in a separate vessel before supplying it to the batch cooking vessel 3 hereby preventing a lowering of the temperature inside the batch cooking vessel 3.

During hydrolysis, hemicellulose is hydrolysed from the lignocellulosic material and forms part of a slurry that comprises the lignocellulosic material and treatment liquor. In order to remove a hydrolysate from the batch cooking vessel 3 a displacement liquor is supplied to the batch cooking vessel 3. The hydrolysate comprises hemicellulose and also other substances that are in liquid form or that are dissolved in liquid of the slurry. Larger particles such as the wood matrix of the hydrolysed lignocellulosic material will be retained inside the treatment vessel.

After displacement of the hydrolysate, an alkaline liquid such as white liquor is supplied to increase the pH value and create alkaline conditions in the treatment vessel. Treatment of the lignocellulosic material is then continued in order to produce pulp as is well known within the art.

According to the invention a batch cooking system 1 for producing pulp is provided. Said batch cooking system 1 comprises a batch cooking vessel 3 and a hydrolysate extracting arrangement 5. Said hydrolysate extracting arrangement 5 is configured to extract hydrolysate in the batch cooking system 1 and the hydrolysate extracting arrangement 5 comprises at least one hydrolysate extraction tank 7 which is connected to a hydrolysate outlet 11 of the batch cooking vessel 3. According to the invention the hydrolysate extracting arrangement further comprises at least one hydrolysate recirculation tank 9 which also is connected to a hydrolysate outlet 11 of the batch cooking vessel 3. In this embodiment the hydrolysate extraction tank 7 and the hydrolysate recirculation tank 9 are connected to the same hydrolysate outlet 11 of the batch cooking vessel. In another embodiment they could however be connected to different hydrolysate outlets. The hydrolysate recirculation tank 9 is also connected to an inlet 13 of the batch cooking vessel 3 such that hydrolysate removed from the batch cooking vessel 3 can be recirculated back to the batch cooking vessel 3 for displacing hydrolysate in a next following batch cooking process.

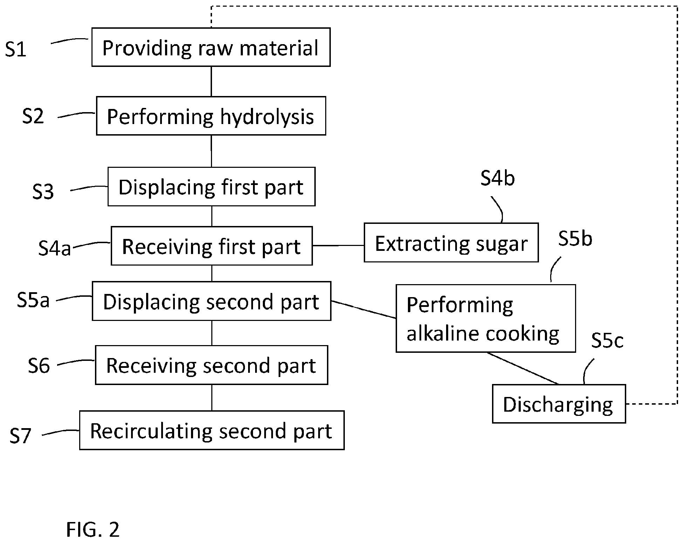

According to the invention a method for extracting hydrolysate in a batch cooking process for producing pulp is also provided. A flow chart of the method is shown in Figure 2 and the steps of the method are described in order below:
S1: Providing lignocellulose raw material to a batch cooking vessel 3.
S2: Performing acid hydrolysis of the lignocellulose raw material in the batch cooking vessel 3. The step of performing acid hydrolysis of the lignocellulose raw material in the batch cooking vessel 3 can comprise steam hydrolysis and/or liquid hydrolysis as described above.
S3: Displacing a first part of a hydrolysate from the batch cooking vessel 3 by adding to the batch cooking vessel 3 at least one first displacement liquid. Said first displacement liquid comprises recycled hydrolysate from a hydrolysate recirculation tank 9. Said recycled hydrolysate has been transferred to the hydrolysate recirculation tank 9 from the batch cooking vessel 3 during a previous batch cooking process. Another first displacement liquid can additionally be used for displacing the first part of the hydrolysate from the batch cooking vessel 3. This displacement liquid could be for example water.
S4a: Receiving the first part of the hydrolysate in at least one hydrolysate extraction tank 7.
S5a: Displacing a second part of the hydrolysate from the batch cooking vessel 3 by adding to the batch cooking vessel 3 at least one second displacement liquid comprising alkaline neutralization liquor, such as for example black liquor or white liquor. Another second displacement liquid can additionally be used for displacing the second part of the hydrolysate from the batch cooking vessel 3. This displacement liquid could be for example water.
S6: Receiving the second part of the hydrolysate in a hydrolysate recirculation tank 9. There could as well be provided more than one hydrolysate recirculation tanks 9 which can be used alternately such that one of the hydrolysate extraction tanks can be cleaned while another is used.
S7: Recirculating said second part of the hydrolysate from the hydrolysate recirculation tank 9 to the batch cooking vessel 3 for displacing a first part of a hydrolysate from the batch cooking vessel 3 in a next batch cooking process performed in the batch cooking vessel 3.

The method can furthermore comprise the step:
S4b: Extracting sugar from the hydrolysate in the hydrolysate extraction tank 7. This step can be performed at any time after step S4a independently of the other steps S5-S7, i.e. after the first part of the hydrolysate has been received in the hydrolysate extraction tank 7.

The further process performed in the batch cooking vessel 3 after the second part of the hydrolysate has been displaced, i.e. after step S5a, comprises the steps:
S5b: Performing alkaline cooking of the material left in the batch cooking vessel 3 after the displacing of the first and second parts of the hydrolysate.
S5c: Discharging the treated material, i.e. the pulp which can be dissolving pulp. Then the process is repeated from step S1, i.e. new raw material is provided into the batch cooking vessel 3, i.e. a new batch of raw material can be treated in the batch cooking vessel 3.

In some embodiments of the invention the batch cooking process is a process for producing dissolving pulp.

The batch cooking system 1 will now be described in more detail with reference to Figure 1.

The hydrolysate extracting arrangement 5 comprises further according to some embodiments of the invention a first hydrolysate removing conduit 15. Said first hydrolysate removing conduit 15 connects a hydrolysate outlet 11 of the batch cooking vessel 3 and the hydrolysate extraction tank 7. The first hydrolysate removing conduit 15 can be one single conduit which is directly connecting the hydrolysate extraction tank 7 and the batch cooking vessel 3 but it can also comprise branched conduits and a conduit part which can be common with a second hydrolysate removing conduit 17 which will be described below. The first hydrolysate removing conduit 15 comprises a first valve 16a.

The hydrolysate extracting arrangement 5 may further comprise a second hydrolysate removing conduit 17 which is connecting a hydrolysate outlet 11 of the batch cooking vessel 3 and an inlet 19a of the at least one hydrolysate recirculation tank 9. The hydrolysate outlet 11 can be the same outlet as the first hydrolysate removing conduit 15 is connected to as shown in Figure 1 but it can also be separate hydrolysate outlets. As described above the first and second hydrolysate removing conduits may comprise a common conduit part which is branched to the separation conduits leading to the hydrolysate extraction tank 7 and the hydrolysate recirculation tank 9 respectively. The second hydrolysate removing conduit 17 comprises a second valve 16b.

The hydrolysate extracting arrangement 5 further comprises at least one recirculation conduit 23 which is connecting an outlet 19b of the at least one hydrolysate recirculation tank 9 with an inlet 13 of the batch cooking vessel 3. A recirculation pump 25 can be connected to the recirculation conduit 23. Hereby hydrolysate received in the hydrolysate recirculation tank 9 can be recirculated back to the batch cooking vessel 3.

The hydrolysate extracting arrangement 5 may further comprise a control system 27 connected to the first and second valves 16a, 16b. The connection may be by wire or wireless. Said control system 27 is configured to control the first and second valves 16a, 16b such that a first part of the hydrolysate which is displaced from the batch cooking vessel 3 is transferred to the hydrolysate extraction tank 7 and a second part of the hydrolysate which is displaced from the batch cooking vessel is transferred to the at least one hydrolysate recirculation tank 9.

The control system 27 may also be connected to the at least one recirculation pump 25. The control system 27 can then be configured to control the recirculation pump 25 such that a content in the hydrolysate recirculation tank 9 is transferred to the batch cooking vessel 3 for displacing the first part of the hydrolysate from the batch cooking vessel 3.

According to some embodiments of the invention the batch cooking system further comprises an alkaline neutralization liquor tank 31 connected to an inlet 14 of the batch cooking vessel 3 via an alkaline neutralization liquor conduit 33 and an alkaline neutralization liquor pump 35 provided in connection with said alkaline neutralization liquor conduit 33. The control system 27 can also be connected to said alkaline neutralization liquor pump 35 and the control system can be configured for controlling said alkaline neutralization liquor pump 35 to add alkaline neutralization liquor from the alkaline neutralization liquor tank 31 into the batch cooking vessel 3 for displacing at least a part of the second part of the hydrolysate.

As discussed above, also other displacement liquors in addition to the first and second displacement liquors can be used for displacing the first and second parts of the hydrolysate. For example water and condensates can be used for the displacing.

The batch cooking vessel 3 is a pressurized vessel and said batch cooking system 1 comprises further a hydrolysis arrangement 21 connected to the batch cooking vessel 3. The hydrolysis arrangement 21 is configured for adding a fluid to the batch cooking vessel 3 for performing a hydrolysis of a lignocellulose raw material provided in the batch cooking vessel 3. The hydrolysis arrangement 21can be configured for adding steam and/or liquid to the batch cooking vessel 3 for performing the hydrolysis.

The batch cooking system 1 can be a system for producing dissolving pulp.

In some embodiments of the invention the hydrolysate extracting arrangement 5 comprises at least two hydrolysate recirculation tanks 9 such that they can be used alternately as described above.

Furthermore according to the invention a hydrolysate extracting arrangement 5 is provided which is configured to be connected to a batch cooking vessel 3 in a batch cooking system 1 for producing pulp as described above. Said hydrolysate extracting arrangement 5 is configured to extract hydrolysate in the batch cooking system 1. The hydrolysate extracting arrangement 5 comprises at least one hydrolysate extraction tank 7 configured to be connected to a hydrolysate outlet 11 of the batch cooking vessel 3 and at least one hydrolysate recirculation tank 9 configured to be connected to a hydrolysate outlet 11 of the batch cooking vessel 3, wherein said hydrolysate recirculation tank 9 also is configured to be connected to an inlet 13 of the batch cooking vessel 3 such that hydrolysate removed from the batch cooking vessel 3 can be recirculated back to the batch cooking vessel 3 for displacing hydrolysate in a next following batch cooking process.

In the embodiment as shown in Figure 1 the hydrolysate extracting arrangement 5 further comprises a first hydrolysate removing conduit 15 which is connected to the hydrolysate extraction tank 7 and which comprises a hydrolysate outlet connector 18, which is configured to be connected to a hydrolysate outlet 11 of the batch cooking vessel 3. Said first hydrolysate removing conduit 15 comprises furthermore a first valve 16a. The hydrolysate extracting arrangement 5 further comprises a second hydrolysate removing conduit 17 which is connected to an inlet 19a of the at least one hydrolysate recirculation tank 9 and which comprises a hydrolysate outlet connector 18, which is configured to be connected to a hydrolysate outlet 11 of the batch cooking vessel 3. The hydrolysate outlet connector 18 of the first and second hydrolysate removing conduits 15, 17 are in this embodiment one and the same connector which is connected to one and the same hydrolysate outlet 11 of the batch cooking vessel. The first and second hydrolysate removing conduits 15, 17 have a common part and are then branched towards the hydrolysate extraction tank 7 and the hydrolysate recirculation tank 9 respectively. The second hydrolysate removing conduit 17 further comprises a second valve 16b. The hydrolysate extracting arrangement 5 further comprises at least one recirculation conduit 23 which is connected to an outlet 19b of the at least one hydrolysate recirculation tank 9 and which comprises a batch cooking vessel inlet connector 20, which is configured to be connected to an inlet 13 of the batch cooking vessel 3. The hydrolysate extracting arrangement 5 further comprises at least one recirculation pump 25 connected to the recirculation conduit and a control system 27 connected to the first and second valves 16a, 16b, wherein said control system 27 is configured to control the first and second valves 16a, 16b such that a first part of the hydrolysate which is displaced from the batch cooking vessel is transferred to the hydrolysate extraction tank 7 and a second part of the hydrolysate which is displaced from the batch cooking vessel is transferred to the at least one hydrolysate recirculation tank 9 when the hydrolysate extracting arrangement 5 is connected to a batch cooking vessel 3 in a batch cooking system 1.

In some embodiments the control system 27 is also connected to the at least one recirculation pump 25 and the control system 27 is configured to control the recirculation pump 25 such that a content in the hydrolysate recirculation tank 9 is transferred to the batch cooking vessel 3 for displacing the first part of the hydrolysate from the batch cooking vessel 3 when the hydrolysate extracting arrangement 5 is connected to a batch cooking vessel 3 in a batch cooking system 1.

In some embodiments of the invention the hydrolysate extracting arrangement 5 comprises at least two hydrolysate recirculation tanks 9 such that they can be used alternately.

## Claims

1. A method for extracting hydrolysate in a batch cooking process in a batch cooking system (1) for producing pulp, said method comprising the steps of:
- providing lignocellulose raw material to a batch cooking vessel (3);
- performing acid hydrolysis of the lignocellulose raw material in the batch cooking vessel (3);
- displacing a first part of a hydrolysate from the batch cooking vessel (3) by adding, by use of a recirculation pump (25) provided in the batch cooking system (1) and controlled by a control system (27) provided in the batch cooking system, to the batch cooking vessel at least one first displacement liquid comprising recycled hydrolysate from a hydrolysate recirculation tank (9), wherein said recycled hydrolysate has been transferred to the hydrolysate recirculation tank (9) from the batch cooking vessel (3) during a previous batch cooking process;
- receiving the first part of the hydrolysate in at least one hydrolysate extraction tank (7) by controlling by the control system (27) a first valve (16a) provided in a first hydrolysate removing conduit (15) which is connecting the hydrolysate extraction tank (7) and the batch cooking vessel (3) in the batch cooking system;
- displacing a second part of the hydrolysate from the batch cooking vessel (3) by adding to the batch cooking vessel (3) at least one second displacement liquid comprising alkaline neutralization liquor; and
- receiving the second part of the hydrolysate in at least one hydrolysate recirculation tank (9) by controlling by the control system (27) a second valve (16b) provided in a second hydrolysate removing conduit (17) which is connecting the hydrolysate recirculation tank (9) and the batch cooking vessel (3) in the batch cooking system.

2. Method according to claim 1, wherein the method comprises the further step:
- recirculating said second part of the hydrolysate from the hydrolysate recirculation tank (9) to the batch cooking vessel (3) for displacing a first part of a hydrolysate from the batch cooking vessel (3) in a next batch cooking process performed in the batch cooking vessel.

3. Method according to claim 1 or 2, wherein the method further comprises the step of extracting sugar from the hydrolysate in the hydrolysate extraction tank (7).

4. Method according to any one of the preceding claims, wherein the method further comprises the step of performing alkaline cooking in the batch cooking vessel (3) after the displacing of the first and second parts of the hydrolysate.

5. Method according to any one of the preceding claims, wherein the batch cooking process is a process for producing dissolving pulp.

6. Method according to any one of the preceding claims, wherein the step of performing acid hydrolysis of the lignocellulose raw material in the batch cooking vessel comprises steam hydrolysis and/or liquid hydrolysis.

7. Method according to any one of the preceding claims, wherein the step of receiving the second part of the hydrolysate in at least one hydrolysate recirculation tank (9) comprises receiving the second part of the hydrolysate in at least two hydrolysate recirculation tanks alternately such that one of the hydrolysate recirculation tanks can be cleaned while the other is used.

8. A batch cooking system (1) for producing pulp, said batch cooking system (1) comprising a batch cooking vessel (3) and a hydrolysate extracting arrangement (5), which hydrolysate extracting arrangement (5) is configured to extract hydrolysate in the batch cooking system (1), whereby the hydrolysate extracting arrangement (5) comprises:
- at least one hydrolysate extraction tank (7) connected to a hydrolysate outlet (11) of the batch cooking vessel (3);
- at least one hydrolysate recirculation tank (9) connected to a hydrolysate outlet (11) of the batch cooking vessel (3), wherein said hydrolysate recirculation tank (9) also is connected to an inlet (13) of the batch cooking vessel (3) such that hydrolysate removed from the batch cooking vessel (3) can be recirculated back to the batch cooking vessel (3) for displacing hydrolysate in a next following batch cooking process;
- a first hydrolysate removing conduit (15) which is connecting a hydrolysate outlet (11) of the batch cooking vessel (3) and the hydrolysate extraction tank (7) and which comprises a first valve (16a);
- a second hydrolysate removing conduit (17) which is connecting a hydrolysate outlet (11) of the batch cooking vessel (3) and an inlet (19a) of the at least one hydrolysate recirculation tank (9) and which comprises a second valve (16b);
- at least one recirculation conduit (23) which is connecting an outlet (19b) of the at least one hydrolysate recirculation tank (9) with an inlet (13) of the batch cooking vessel (3);
- at least one recirculation pump (25) connected to the recirculation conduit; and
- a control system (27) connected to the first and second valves (16a, 16b), wherein said control system (27) is configured to control the first and second valves (16a, 16b) such that a first part of the hydrolysate which is displaced from the batch cooking vessel is transferred to the hydrolysate extraction tank (7) and a second part of the hydrolysate which is displaced from the batch cooking vessel is transferred to the at least one hydrolysate recirculation tank (9), wherein the control system (27) also is connected to the at least one recirculation pump (25) and wherein the control system (27) is configured to control the recirculation pump (25) such that a content in the hydrolysate recirculation tank (9) is transferred to the batch cooking vessel (3) for displacing the first part of the hydrolysate from the batch cooking vessel (3).

9. Batch cooking system according to claim 8, wherein said batch cooking system further comprises an alkaline neutralization liquor tank (31) connected to an inlet (14) of the batch cooking vessel (3) via an alkaline neutralization liquor conduit (33) and an alkaline neutralization liquor pump (35) provided in connection with said alkaline neutralization liquor conduit (33), wherein the control system (27) further is connected to said alkaline neutralization liquor pump (35) and is configured for controlling said alkaline neutralization liquor pump (35) to add alkaline neutralization liquor from the alkaline neutralization liquor tank (31) into the batch cooking vessel (3) for displacing at least a part of the second part of the hydrolysate.

10. Batch cooking system according to any one of the claims 8-9, wherein said batch cooking vessel (3) is a pressurized vessel and wherein said batch cooking system (1) further comprises a hydrolysis arrangement (21) connected to the batch cooking vessel (3), which hydrolysis arrangement (21) is configured for adding a fluid to the batch cooking vessel (3) for performing a hydrolysis of a lignocellulose raw material provided in the batch cooking vessel (3).

11. Batch cooking system according to claim 10, wherein the hydrolysis arrangement (21) is configured for adding steam and/or liquid to the batch cooking vessel (3) for performing the hydrolysis.

12. Batch cooking system according to any one of the claims 8-11, wherein the batch cooking system (1) is a system for producing dissolving pulp.

13. Batch cooking system according to any one of the claims 8-12, wherein the hydrolysate extracting arrangement (5) comprises at least two hydrolysate recirculation tanks (9) such that they can be used alternately.

14. A hydrolysate extracting arrangement for use in a batch cooking system for producing pulp according to any one of the claims 8-13, said hydrolysate extracting arrangement being configured to be connected to the batch cooking vessel (3) ofthe batch cooking system (1), said hydrolysate extracting arrangement (5) being configured to extract hydrolysate in the batch cooking system (1), whereby the hydrolysate extracting arrangement (5) comprises:
- at least one hydrolysate extraction tank (7) configured to be connected to a hydrolysate outlet (11) of the batch cooking vessel (3);
- at least one hydrolysate recirculation tank (9) configured to be connected to a hydrolysate outlet (11) of the batch cooking vessel (3), wherein said hydrolysate recirculation tank (9) also is configured to be connected to an inlet (13) of the batch cooking vessel (3) such that hydrolysate removed from the batch cooking vessel (3) can be recirculated back to the batch cooking vessel (3) for displacing hydrolysate in a next following batch cooking process;
- a first hydrolysate removing conduit (15) which is connected to the hydrolysate extraction tank (7) and which comprises a hydrolysate outlet connector (18), which is configured to be connected to a hydrolysate outlet (11) of the batch cooking vessel (3), said first hydrolysate removing conduit (15) further comprising a first valve (16a);
- a second hydrolysate removing conduit (17) which is connected to an inlet (19a) of the at least one hydrolysate recirculation tank (9) and which comprises a hydrolysate outlet connector (18), which is configured to be connected to a hydrolysate outlet (11) of the batch cooking vessel (3), said second hydrolysate removing conduit (17) further comprising a second valve (16b);
- at least one recirculation conduit (23) which is connected to an outlet (19b) of the at least one hydrolysate recirculation tank (9) and which comprises a batch cooking vessel inlet connector (20), which is configured to be connected to an inlet (13) of the batch cooking vessel (3);
- at least one recirculation pump (25) connected to the recirculation conduit; and
- a control system (27) connected to the first and second valves (16a, 16b), wherein said control system (27) is configured to control the first and second valves (16a, 16b) such that a first part of the hydrolysate which is displaced from the batch cooking vessel is transferred to the hydrolysate extraction tank (7) and a second part of the hydrolysate which is displaced from the batch cooking vessel is transferred to the at least one hydrolysate recirculation tank (9) when the hydrolysate extracting arrangement (5) is connected to a batch cooking vessel (3) in a batch cooking system (1), wherein the control system (27) also is connected to the at least one recirculation pump (25) and wherein the control system (27) is configured to control the recirculation pump (25) such that a content in the hydrolysate recirculation tank (9) is transferred to the batch cooking vessel (3) for displacing the first part of the hydrolysate from the batch cooking vessel (3) when the hydrolysate extracting arrangement (5) is connected to a batch cooking vessel (3) in a batch cooking system (1).

15. Hydrolysate extracting arrangement (5) according to claim 14, wherein the hydrolysate extracting arrangement (5) comprises at least two hydrolysate recirculation tanks (9) such that they can be used alternately.

## Patentansprüche

1. Verfahren zum Extrahieren von Hydrolysat in einem Batch-Kochprozess in einem Batch-Kochsystem (1) zum Herstellen von Zellstoff, das Verfahren umfassend die Schritte:
- Bereitstellen von Lignocellulose-Rohmaterial zu einem Batch-Kochgefäß (3);
- Durchführen einer sauren Hydrolyse des Lignocellulose-Rohmaterials in dem Batch-Kochgefäß (3);
- Verdrängen eines ersten Teils eines Hydrolysats aus dem Batch-Kochgefäß (3) durch ein Zugeben, unter Verwendung einer Umwälzpumpe (25), die in dem Batch-Kochsystem (1) bereitgestellt und durch ein Steuersystem (27) gesteuert wird, das in dem Batch-Kochsystem bereitgestellt ist, zu dem Batch-Kochgefäß mindestens einer ersten Verdrängungsflüssigkeit, umfassend recyceltes Hydrolysat aus einem Hydrolysat-Umwälztank (9), wobei das recycelte Hydrolysat während eines vorherigen Batch-Kochprozess zu dem Hydrolysat-Umwälztank (9) von dem Batch-Kochgefäß (3) übertragen worden ist;
- Aufnehmen des ersten Teils des Hydrolysats in mindestens einem Hydrolysat-Extraktionstank (7) durch das Steuern durch das Steuersystem (27) eines ersten Ventils (16a), das in einer ersten Hydrolysat-Entfernungsleitung (15) bereitgestellt ist, die den Hydrolysat-Extraktionstank (7) und das Batch-Kochgefäß (3) in dem Batch-Kochsystem verbindet;
- Verdrängen eines zweiten Teils des Hydrolysats aus dem Batch-Kochgefäß (3) durch das Zugeben von mindestens einer zweiten Verdrängungsflüssigkeit, umfassend alkalische Neutralisationslauge, zu dem Batch-Kochgefäß (3); und
- Aufnehmen des zweiten Teils des Hydrolysats in mindestens einem Hydrolysat-Umwälztank (9) durch das Steuern durch das Steuersystem (27) eines zweiten Ventils (16b), das in einer zweiten Hydrolysat-Entfernungsleitung (17) bereitgestellt ist, die den Hydrolysat-Umwälztank (9) und das Batch-Kochgefäß (3) in dem Batch-Kochsystem verbindet.

2. Verfahren nach Anspruch 1, wobei das Verfahren den weiteren Schritt umfasst:
- Umwälzen des zweiten Teils des Hydrolysats aus dem Hydrolysat-Umwälztank (9) zu dem Batch-Kochgefäß (3) zum Verdrängen eines ersten Teils eines Hydrolysats aus dem Batch-Kochgefäß (3) in einem nächsten Batch-Kochprozess, der in dem Batch-Kochgefäß durchgeführt wird.

3. Verfahren nach Anspruch 1 oder 2, wobei das Verfahren ferner den Schritt des Extrahierens von Zucker aus dem Hydrolysat in dem Hydrolysat-Extraktionstank (7) umfasst.

4. Verfahren nach einem der vorstehenden Ansprüche, wobei das Verfahren ferner den Schritt des Durchführens von alkalischem Kochen in dem Batch-Kochgefäß (3) nach dem Verdrängen des ersten und des zweiten Teils des Hydrolysats umfasst.

5. Verfahren nach einem der vorstehenden Ansprüche, wobei der Batch-Kochprozess ein Prozess zum Herstellen von Chemiezellstoff ist.

6. Verfahren nach einem der vorstehenden Ansprüche, wobei der Schritt des Durchführens einer Säurehydrolyse des Lignocellulose-Rohmaterials in dem Batch-Kochgefäß Dampfhydrolyse und/oder Flüssighydrolyse umfasst.

7. Verfahren nach einem der vorstehenden Ansprüche, wobei der Schritt des Aufnehmens des zweiten Teils des Hydrolysats in mindestens einem Hydrolysat-Umwälztank (9) das Aufnehmen des zweiten Teils des Hydrolysats in mindestens zwei Hydrolysat-Umwälztanks derart abwechselnd umfasst, dass einer der Hydrolysat-Umwälztanks gereinigt werden kann, während der andere verwendet wird.

8. Batch-Kochsystem (1) zum Herstellen von Zellstoff, das Batch-Kochsystem (1) umfassend ein Batch-Kochgefäß (3) und eine Hydrolysat-Extrahieranordnung (5), wobei die Hydrolysat-Extrahieranordnung (5) konfiguriert ist, um Hydrolysat in dem Batch-Kochsystem (1) zu extrahieren, wobei die Hydrolysat-Extrahieranordnung (5) umfasst:
- mindestens einen Hydrolysat-Extraktionstank (7), der mit einem Hydrolysat-Auslass (11) des Batch-Kochgefäßes (3) verbunden ist;
- mindestens einen Hydrolysat-Umwälztank (9), der mit einem Hydrolysat-Auslass (11) des Batch-Kochgefäßes (3) verbunden ist, wobei der Hydrolysat-Umwälztank (9) ebenso mit einem Einlass (13) des Batch-Kochgefäßes (3) derart verbunden ist, dass aus dem Batch-Kochgefäß (3) entferntes Hydrolysat zurück zu dem Batch-Kochgefäß (3) umgewälzt werden kann, zum Verdrängen von Hydrolysat in einem nächstfolgenden Batch-Kochprozess;
- eine erste Hydrolysat-Entfernungsleitung (15), die einen Hydrolysat-Auslass (11) des Batch-Kochgefäßes (3) und den Hydrolysat-Extraktionstank (7) verbindet und die ein erstes Ventil (16a) umfasst;
- eine zweite Hydrolysat-Entfernungsleitung (17), die einen Hydrolysat-Auslass (11) des Batch-Kochgefäßes (3) und einen Einlass (19a) des mindestens einen Hydrolysat-Umwälztanks (9) verbindet und die ein zweites Ventil (16b) umfasst;
- mindestens eine Umwälzleitung (23), die einen Auslass (19b) des mindestens einen Hydrolysat-Umwälztanks (9) mit einem Einlass (13) des Batch-Kochgefäßes (3) verbindet;
- mindestens eine Umwälzpumpe (25), die mit der Umwälzleitung verbunden ist; und
- ein Steuersystem (27), das mit den ersten und dem zweiten Ventil (16a, 16b) verbunden ist, wobei das Steuersystem (27) konfiguriert ist, um das erste und das zweite Ventil (16a, 16b) derart zu steuern, dass ein erster Teil des Hydrolysats, der aus dem Batch-Kochgefäß verdrängt wird, zu dem Hydrolysat-Extraktionstank (7) übertragen wird und ein zweiter Teil des Hydrolysats, der aus dem Batch-Kochgefäß verdrängt wird, zu dem mindestens einen Hydrolysat-Umwälztank (9) übertragen wird, wobei das Steuersystem (27) ebenso mit der mindestens einen Umwälzpumpe (25) verbunden ist und wobei das Steuersystem (27) konfiguriert ist, um die Umwälzpumpe (25) derart zu steuern, dass ein Inhalt in dem Hydrolysat-Umwälztank (9) zu dem Batch-Kochgefäß (3) übertragen wird, zum Verdrängen des ersten Teils des Hydrolysats aus dem Batch-Kochgefäß (3).

9. Batch-Kochsystem nach Anspruch 8, wobei das Batch-Kochsystem ferner einen alkalischen Neutralisationslaugentank (31) umfasst, der mit einem Einlass (14) des Batch-Kochgefäßes (3) über eine alkalische Neutralisationslaugenleitung (33) und eine alkalische Neutralisationslaugenpumpe (35) verbunden ist, die in Verbindung mit der alkalischen Neutralisationslaugenleitung (33) bereitgestellt ist, wobei das Steuersystem (27) ferner mit der alkalischen Neutralisationslaugenpumpe (35) verbunden ist und konfiguriert ist, um die alkalische Neutralisationslaugenpumpe (35) zu steuern, um alkalische Neutralisationslauge aus dem alkalischen Neutralisationslaugentank (31) in das Batch-Kochgefäß (3) zuzugeben, um mindestens einen Teil des zweiten Teils des Hydrolysats zu verdrängen.

10. Batch-Kochsystem nach einem der Ansprüche 8 bis 9, wobei das Batch-Kochgefäß (3) ein Druckgefäß ist und wobei das Batch-Kochsystem (1) ferner eine Hydrolyseanordnung (21) umfasst, die mit dem Batch-Kochgefäß (3) verbunden ist, wobei die Hydrolyseanordnung (21) konfiguriert ist, um ein Fluid zu dem Batch-Kochgefäß (3) zuzugeben, zum Durchführen einer Hydrolyse eines Lignocellulose-Rohmaterials, das in dem Batch-Kochgefäß (3) bereitgestellt ist.

11. Batch-Kochsystem nach Anspruch 10, wobei die Hydrolyseanordnung (21) zum Zugeben von Dampf und/oder Flüssigkeit zu dem Batch-Kochgefäß (3) zum Durchführen der Hydrolyse konfiguriert ist.

12. Batch-Kochsystem nach einem der Ansprüche 8 bis 11, wobei das Batch-Kochsystem (1) ein System zum Herstellen von Chemiezellstoff ist.

13. Batch-Kochsystem nach einem der Ansprüche 8 bis 12, wobei die Hydrolysat-Extrahieranordnung (5) mindestens zwei Hydrolysat-Umwälztanks (9) derart umfasst, dass sie abwechselnd verwendet werden können.

14. Hydrolysat-Extrahieranordnung zur Verwendung in einem Batch-Kochsystem zum Herstellen von Zellstoff nach einem der Ansprüche 8 bis 13, wobei die Hydrolysat-Extrahieranordnung konfiguriert ist, um mit dem Batch-Kochgefäß (3) des Batch-Kochsystems (1) verbunden zu werden, wobei die Hydrolysat-Extraktionsanordnung (5) konfiguriert ist, um Hydrolysat in dem Batch-Kochsystem (1) zu extrahieren, wobei die Hydrolysat-Extrahieranordnung (5) umfasst:
- mindestens einen Hydrolysat-Extraktionstank (7), der konfiguriert ist, um mit einem Hydrolysat-Auslass (11) des Batch-Kochgefäßes (3) verbunden zu werden;
- mindestens einen Hydrolysat-Umwälztank (9), der konfiguriert ist, um mit einem Hydrolysat-Auslass (11) des Batch-Kochgefäßes (3) verbunden zu werden, wobei der Hydrolysat-Umwälztank (9) ebenso konfiguriert ist, um mit einem Einlass (13) des Batch-Kochgefäßes (3) derart verbunden zu werden, dass Hydrolysat, das aus dem Batch-Kochgefäß (3) entfernt wird, zurück zu dem Batch-Kochgefäß (3) umgewälzt werden kann, zum Verdrängen des Hydrolysats in einem nächstfolgenden Batch-Kochprozess;
- eine erste Hydrolysat-Entfernungsleitung (15), die mit dem Hydrolysat-Extraktionstank (7) verbunden ist und die einen Hydrolysat-Auslassverbinder (18) umfasst, der konfiguriert ist, um mit einem Hydrolysat-Auslass (11) des Batch-Kochgefäßes (3) verbunden zu werden, die erste Hydrolysat-Entfernungsleitung (15) ferner umfassend ein erstes Ventil (16a);
- eine zweite Hydrolysat-Entfernungsleitung (17), die mit einem Einlass (19a) des mindestens einen Hydrolysat-Umwälztanks (9) verbunden ist und die einen Hydrolysat-Auslassverbinder (18) umfasst, der konfiguriert ist, um mit einem Hydrolysat-Auslass (11) des Batch-Kochgefäßes (3) verbunden zu werden, die zweite Hydrolysat-Entfernungsleitung (17) ferner umfassend ein zweites Ventil (16b);
- mindestens eine Umwälzleitung (23), die mit einem Auslass (19b) des mindestens einen Hydrolysat-Umwälztanks (9) verbunden ist und die einen Batch-Kochgefäß-Einlassverbinder (20) umfasst, der konfiguriert ist, um mit einem Einlass (13) des Batch-Kochgefäßes (3) verbunden zu werden;
- mindestens eine Umwälzpumpe (25), die mit der Umwälzleitung verbunden ist; und
- ein Steuersystem (27), das mit dem ersten und dem zweiten Ventil (16a, 16b) verbunden ist, wobei das Steuersystem (27) konfiguriert ist, um das erste und das zweite Ventil (16a, 16b) derart zu steuern, dass ein erster Teil des Hydrolysats, der aus dem Batch-Kochgefäß verdrängt wird, zu dem Hydrolysat-Extraktionstank (7) übertragen wird und ein zweiter Teil des Hydrolysats, der aus dem Batch-Kochgefäß verdrängt wird, zu dem mindestens einen Hydrolysat-Umwälztank (9) übertragen wird, wenn die Hydrolysat-Extrahieranordnung (5) mit einem Batch-Kochgefäß (3) in einem Batch-Kochsystem (1) verbunden ist, wobei das Steuersystem (27) ebenso mit der mindestens einen Umwälzpumpe (25) verbunden ist und wobei das Steuersystem (27) konfiguriert ist, um die Umwälzpumpe (25) derart zu steuern, dass ein Inhalt in dem Hydrolysat-Umwälztank (9) zu dem Batch-Kochgefäß (3) übertragen wird, zum Verdrängen des ersten Teils des Hydrolysats aus dem Batch-Kochgefäß (3), wenn die Hydrolysat-Extrahieranordnung (5) mit einem Batch-Kochgefäß (3) in einem Batch-Kochsystem (1) verbunden ist.

15. Hydrolysat-Extrahieranordnung (5) nach Anspruch 14, wobei die Hydrolysat-Extrahieranordnung (5) mindestens zwei Hydrolysat-Umwälztanks (9) derart umfasst, dass sie abwechselnd verwendet werden können.

## Revendications

1. Procédé permettant d'extraire un hydrolysat dans un processus de cuisson par lots dans un système de cuisson par lots (1) permettant de produire de la pâte, ledit procédé comprenant les étapes consistant à :
- fournir une matière première de lignocellulose à une cuve de cuisson par lots (3) ;
- mettre en œuvre une hydrolyse acide de la matière première de lignocellulose dans la cuve de cuisson par lots (3) ;
- déplacer une première partie d'un hydrolysat hors de la cuve de cuisson par lots (3) en ajoutant, par l'utilisation d'une pompe de recirculation (25) fournie dans le système de cuisson par lots (1) et commandée par un système de commande (27) fourni dans le système de cuisson par lots, à la cuve de cuisson par lots au moins un premier liquide de déplacement comprenant un hydrolysat recyclé provenant d'un réservoir de recirculation d'hydrolysat (9), dans lequel ledit hydrolysat recyclé a été transféré vers le réservoir de recirculation d'hydrolysat (9) à partir de la cuve de cuisson par lots (3) pendant un processus de cuisson par lots précédent ;
- recevoir la première partie de l'hydrolysat dans au moins un réservoir d'extraction d'hydrolysat (7) en commandant par le système de commande (27) une première vanne (16a) fournie dans un premier conduit de retrait d'hydrolysat (15) qui raccorde le réservoir d'extraction d'hydrolysat (7) et la cuve de cuisson par lots (3) dans le système de cuisson par lots ;
- déplacer une seconde partie de l'hydrolysat hors de la cuve de cuisson par lots (3) en ajoutant à la cuve de cuisson par lots (3) au moins un second liquide de déplacement comprenant une liqueur de neutralisation alcaline ; et
- recevoir la seconde partie de l'hydrolysat dans au moins un réservoir de recirculation d'hydrolysat (9) en commandant par le système de commande (27) une seconde vanne (16b) fournie dans un second conduit de retrait d'hydrolysat (17) qui raccorde le réservoir de recirculation d'hydrolysat (9) et la cuve de cuisson par lots (3) dans le système de cuisson par lots.

2. Procédé selon la revendication 1, dans lequel le procédé comprend l'étape supplémentaire :
- remise en circulation de ladite seconde partie de l'hydrolysat provenant du réservoir de recirculation d'hydrolysat (9) vers la cuve de cuisson par lots (3) pour le déplacement d'une première partie d'un hydrolysat hors de la cuve de cuisson par lots (3) dans un processus de cuisson par lots suivant mis en œuvre dans la cuve de cuisson par lots.

3. Procédé selon la revendication 1 ou 2, dans lequel le procédé comprend en outre l'étape consistant à extraire du sucre de l'hydrolysat dans le réservoir d'extraction d'hydrolysat (7).

4. Procédé selon l'une quelconque des revendications précédentes, dans lequel le procédé comprend en outre l'étape de mise en œuvre d'une cuisson alcaline dans la cuve de cuisson par lots (3) après le déplacement des première et seconde parties de l'hydrolysat.

5. Procédé selon l'une quelconque des revendications précédentes, dans lequel le processus de cuisson par lots est un processus permettant de produire de la pâte dissolvante.

6. Procédé selon l'une quelconque des revendications précédentes, dans lequel l'étape de mise en œuvre d'une hydrolyse acide de la matière première de lignocellulose dans la cuve de cuisson par lots comprend une hydrolyse à la vapeur et/ou une hydrolyse liquide.

7. Procédé selon l'une quelconque des revendications précédentes, dans lequel l'étape de réception de la seconde partie de l'hydrolysat dans au moins un réservoir de recirculation d'hydrolysat (9) comprend la réception de la seconde partie de l'hydrolysat dans au moins deux réservoirs de recirculation d'hydrolysat en alternance de telle sorte que l'un des réservoirs de recirculation d'hydrolysat peut être nettoyé alors que l'autre est utilisé.

8. Système de cuisson par lots (1) permettant de produire de la pâte, ledit système de cuisson par lots (1) comprenant une cuve de cuisson par lots (3) et un agencement d'extraction d'hydrolysat (5), cet agencement d'extraction d'hydrolysat (5) étant configuré pour extraire un hydrolysat dans le système de cuisson par lots (1), moyennant quoi l'agencement d'extraction d'hydrolysat (5) comprend :
- au moins un réservoir d'extraction d'hydrolysat (7) raccordé à une sortie d'hydrolysat (11) de la cuve de cuisson par lots (3) ;
- au moins un réservoir de recirculation d'hydrolysat (9) raccordé à une sortie d'hydrolysat (11) de la cuve de cuisson par lots (3), dans lequel ledit réservoir de recirculation d'hydrolysat (9) est également raccordé à une entrée (13) de la cuve de cuisson par lots (3) de telle sorte que l'hydrolysat retiré de la cuve de cuisson par lots (3) peut être remis en circulation vers la cuve de cuisson par lots (3) pour un déplacement de l'hydrolysat dans un processus de cuisson par lots suivant ;
- un premier conduit de retrait d'hydrolysat (15) qui raccorde une sortie d'hydrolysat (11) de la cuve de cuisson par lots (3) et le réservoir d'extraction d'hydrolysat (7) et qui comprend une première vanne (16a) ;
- un second conduit de retrait d'hydrolysat (17) qui raccorde une sortie d'hydrolysat (11) de la cuve de cuisson par lots (3) et une entrée (19a) de l'au moins un réservoir de recirculation d'hydrolysat (9) et qui comprend une seconde vanne (16b) ;
- au moins un conduit de recirculation (23) qui raccorde une sortie (19b) de l'au moins un réservoir de recirculation d'hydrolysat (9) à une entrée (13) de la cuve de cuisson par lots (3) ;
- au moins une pompe de recirculation (25) raccordée au conduit de recirculation ; et
- un système de commande (27) connecté aux première et seconde vannes (16a, 16b), dans lequel ledit système de commande (27) est configuré pour commander les première et seconde vannes (16a, 16b) de telle sorte qu'une première partie de l'hydrolysat qui est déplacée hors de la cuve de cuisson par lots est transférée vers le réservoir d'extraction d'hydrolysat (7) et une seconde partie de l'hydrolysat qui est déplacée hors de la cuve de cuisson par lots est transférée à l'au moins un réservoir de recirculation d'hydrolysat (9), dans lequel le système de commande (27) est également connecté à l'au moins une pompe de recirculation (25) et dans lequel le système de commande (27) est configuré pour commander la pompe de recirculation (25) de telle sorte qu'un contenu dans le réservoir de recirculation d'hydrolysat (9) est transféré à la cuve de cuisson par lots (3) pour un déplacement de la première partie de l'hydrolysat hors de la cuve de cuisson par lots (3).

9. Système de cuisson par lots selon la revendication 8, dans lequel ledit système de cuisson par lots comprend en outre un réservoir de liqueur de neutralisation alcaline (31) raccordé à une entrée (14) de la cuve de cuisson par lots (3) par l'intermédiaire d'un conduit de liqueur de neutralisation alcaline (33) et d'une pompe à liqueur de neutralisation alcaline (35) fournie en raccordement avec le conduit de liqueur de neutralisation alcaline (33), dans lequel le système de commande (27) est connecté en outre à ladite pompe à liqueur de neutralisation alcaline (35) et est configuré pour commander ladite pompe à liqueur de neutralisation alcaline (35) pour ajouter une liqueur de neutralisation alcaline provenant du réservoir de liqueur de neutralisation alcaline (31) dans la cuve de cuisson par lots (3) pour le déplacement d'au moins une partie de la seconde partie de l'hydrolysat.

10. Système de cuisson par lots selon l'une quelconque des revendications 8 à 9, dans lequel ladite cuve de cuisson par lots (3) est une cuve sous pression et dans lequel ledit système de cuisson par lots (1) comprend en outre un agencement d'hydrolyse (21) raccordé à la cuve de cuisson par lots (3), cet agencement d'hydrolyse (21) étant conçu pour l'ajout d'un fluide à la cuve de cuisson par lots (3) pour la mise en œuvre d'une hydrolyse d'une matière première de lignocellulose fournie dans la cuve de cuisson par lots (3).

11. Système de cuisson par lots selon la revendication 10, dans lequel l'agencement d'hydrolyse (21) est conçu pour l'ajout de vapeur et/ou de liquide à la cuve de cuisson par lots (3) pour la mise en œuvre de l'hydrolyse.

12. Système de cuisson par lots selon l'une quelconque des revendications 8 à 11, dans lequel le système de cuisson par lots (1) est un système permettant de produire de la pâte dissolvante.

13. Système de cuisson par lots selon l'une quelconque des revendications 8 à 12, dans lequel l'agencement d'extraction d'hydrolysat (5) comprend au moins deux réservoirs de recirculation d'hydrolysat (9) de telle sorte qu'ils peuvent être utilisés en alternance.

14. Agencement d'extraction d'hydrolysat pour utilisation dans un système de cuisson par lots permettant de produire de la pâte selon l'une quelconque des revendications 8 à 13, ledit agencement d'extraction d'hydrolysat étant conçu pour être raccordé à la cuve de cuisson par lots (3) du système de cuisson par lots (1), ledit agencement d'extraction d'hydrolysat (5) étant conçu pour extraire un hydrolysat dans le système de cuisson par lots (1), moyennant quoi l'agencement d'extraction d'hydrolysat (5) comprend :
- au moins un réservoir d'extraction d'hydrolysat (7) conçu pour être raccordé à une sortie d'hydrolysat (11) de la cuve de cuisson par lots (3) ;
- au moins un réservoir de recirculation d'hydrolysat (9) conçu pour être raccordé à une sortie d'hydrolysat (11) de la cuve de cuisson par lots (3), dans lequel ledit réservoir de recirculation d'hydrolysat (9) est conçu également pour être raccordé à une entrée (13) de la cuve de cuisson par lots (3) de telle sorte que l'hydrolysat retiré de la cuve de cuisson par lots (3) peut être remis en circulation vers la cuve de cuisson par lots (3) pour un déplacement de l'hydrolysat dans un processus de cuisson par lots suivant ;
- un premier conduit de retrait d'hydrolysat (15) qui est raccordé au réservoir d'extraction d'hydrolysat (7) et qui comprend un raccord de sortie d'hydrolysat (18), qui est conçu pour être raccordé à une sortie d'hydrolysat (11) de la cuve de cuisson par lots (3), ledit premier conduit de retrait d'hydrolysat (15) comprenant en outre une première vanne (16a) ;
- un second conduit de retrait d'hydrolysat (17) qui est raccordé à une entrée (19a) de l'au moins un réservoir de recirculation d'hydrolysat (9) et qui comprend un raccord de sortie d'hydrolysat (18), qui est conçu pour être raccordé à une sortie d'hydrolysat (11) de la cuve de cuisson par lots (3), ledit second conduit de retrait d'hydrolysat (17) comprenant en outre une seconde vanne (16b) ;
- au moins un conduit de recirculation (23) qui est raccordé à une sortie (19b) de l'au moins un réservoir de recirculation d'hydrolysat (9) et qui comprend un raccord d'entrée (20) de cuve de cuisson par lots, qui est conçu pour être raccordé à une entrée (13) de la cuve de cuisson par lots (3) ;
- au moins une pompe de recirculation (25) raccordée au conduit de recirculation ; et
- un système de commande (27) connecté aux première et seconde vannes (16a, 16b), dans lequel ledit système de commande (27) est configuré pour commander les première et seconde vannes (16a, 16b) de telle sorte qu'une première partie de l'hydrolysat qui est déplacée hors de la cuve de cuisson par lots est transférée au réservoir d'extraction d'hydrolysat (7) et une seconde partie de l'hydrolysat qui est déplacée hors de la cuve de cuisson par lots est transférée à l'au moins un réservoir de recirculation d'hydrolysat (9) lorsque l'agencement d'extraction d'hydrolysat (5) est raccordé à une cuve de cuisson par lots (3) dans un système de cuisson par lots (1), dans lequel le système de commande (27) est également raccordé à l'au moins une pompe de recirculation (25) et dans lequel le système de commande (27) est configuré pour commander la pompe de recirculation (25) de telle sorte qu'un contenu dans le réservoir de recirculation d'hydrolysat (9) est transféré à la cuve de cuisson par lots (3) pour un déplacement de la première partie de l'hydrolysat hors de la cuve de cuisson par lots (3) lorsque l'agencement d'extraction d'hydrolysat (5) est raccordé à une cuve de cuisson par lots (3) dans un système de cuisson par lots (1).

15. Agencement d'extraction d'hydrolysat (5) selon la revendication 14, dans lequel l'agencement d'extraction d'hydrolysat (5) comprend au moins deux réservoirs de recirculation d'hydrolysat (9) de telle sorte qu'ils peuvent être utilisés en alternance.
